# EUROPEAN PATENT APPLICATION

(11) **EP 3 501 505 A1**
(43) Date of publication of application: **26.06.2019**
(21) Application number: 18020649.2
(22) Date of filing: 20.12.2018
(51) Int. Cl.: A61K 9/20, A61K 9/48, A61K 31/506

(54) **A DRUG FORM COMPRISING CRYSTALLINE NILOTINIB**

(30) Priority: 20.12.2017 CZ 20170821
(71) Applicant: Zentiva K.S., 102 37 Praha 10 (CZ)
(72) Inventor: Kovacik, Pavel, 588 56 Telc (CZ)
(74) Representative: Jirotkova, Ivana

(57) **Abstract**

The present solution relates to a crystalline dispersion of nilotinib hydrochloride in a polymeric matrix, its use for the preparation of a drug form and a preparation method of this dispersion using the hot melt extrusion method.

## Description

### Field of the Invention

The present invention relates to a crystalline dispersion of nilotinib hydrochloride in a polymeric matrix, its use for the preparation of a drug form and a preparation method of this dispersion using the hot melt extrusion method.

### Background Art

The active ingredient, systematically 4-methyl-N-[3-(4-methylimidazol-1-yl)-5-(trifluoromethyl)phenyl]-3-[(4-pyridin-3-ylpyrimidin-2-yl)amino]benzamide of formula 1 is a selective tyrosine kinase inhibitor of a new generation. Nilotinib inhibits the activity of the Bcr-Abl oncoprotein, which causes Philadelphia-positive (Ph+) chronic myeloid leukaemia (CML) and Philadelphia-positive (Ph+) acute lymphoblastic leukaemia (ALL).

The molecule of nilotinib, which was first described in the international patent application WO2004005281, has the following structural formula:

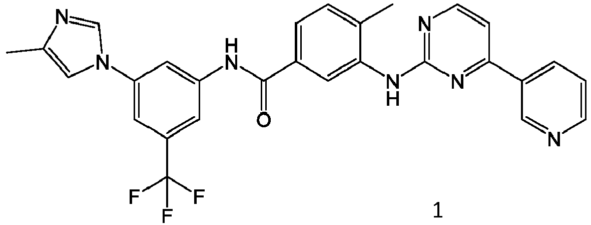

Depending on the preparation conditions, nilotinib can produce various kinds of crystalline forms or an amorphous form. These forms differ from each other with their crystalline arrangement and their physical characteristics, especially solubility and bioavailability. The patent applications WO2007015870, WO2007015871, WO2010054056, WO2011163222, WO2012070062, WO2014059518 and WO2014174456 describe various crystalline forms of nilotinib and its salts.

The original preparation, sold under the trade name Tasigna, comprises crystalline nilotinib hydrochloride of form B, which is the monohydrate of the said salt, which was first described in the patent application WO2007015870. The original preparation Tasigna is available in the market in a drug form with the total strength of 150 and 200 mg of the active ingredient with the recommended daily dosage of twice a day. The drug form is represented by gelatin capsules with immediate release of the active ingredient. According to the biopharmaceutical classification system, nilotinib belongs with its assessment to the BCS II group of substances, i.e. substances that are well absorbable, but poorly soluble. Polymorphism plays an important role with such substances. Individual forms of nilotinib hydrochloride significantly differ from each other with their solubility.

The patent application WO2010054056 describes the crystalline forms T1 to T19 of nilotinib hydrochloride. The form T17 is described as an anhydrous form of nilotinib hydrochloride. The application WO2012164578 describes and mentions problems with conversion of various forms of nilotinib on exposure to an aqueous environment or moisture. The applications WO2012164578 and WO2013074432 deal with problems that are caused by the preparation of nilotinib in a formulation, such as the lack of compatibility with excipients and the impossibility to use some technologies, as e.g. wet granulation. The patent application WO2017064538 also deals with instability of the form T17 and its conversion to nilotinib hydrochloride dihydrate. The application shows how dry granulation and suitable excipients can be used to prevent conversion of the form T17 to other forms during the preparation of the final drug form as well as during the life cycle of the preparation. However, no information is mentioned about the dissolution and whether the required dissolution profile can be achieved with such a formulation that would be comparable to the original preparation Tasigna.

The patent application WO2008037716 describes solid oral pharmaceutical compositions of nilotinib with common pharmaceutical excipients. These pharmaceutical compositions are prepared by wet granulation and the granulate is subsequently filled into capsules. In the application, the key excipient is the surfactant Poloxamer 188, which supports dissolution of the active ingredient. The patent application WO2009100176 describes solid dispersions comprising tyrosine kinase inhibitors and a method of their preparation. The patent application WO2012164578 describes pharmaceutical compositions of nilotinib prepared by dry granulation or by mixing nilotinib with excipients and filling the powder mixture prepared this way into a capsule. The patent application WO2012174082 describes solubilized or amorphous pharmaceutical compositions of nilotinib comprising and organic acid as a solubilizing agent. The patent application WO2013074432 describes a coated tablet comprising nilotinib in the core and a polymeric coating, the degradation of this composition being 4-15 minutes delayed.

In the art, there is still a need of an easy, economical preparation method of a pharmaceutical formulation comprising crystalline nilotinib hydrochloride in the T17 form with good solubility and stability, which has not been successfully found yet.

### Disclosure of the Invention

An object of the invention is a crystalline dispersion of nilotinib hydrochloride in the polymorphic form T17 in a polymeric matrix. Another object of the invention is a preparation method of the crystalline dispersion of nilotinib hydrochloride in the polymorphic form T17 in a polymeric matrix wherein nilotinib hydrochloride of form T17 is mixed with a polymer and the resulting mixture is subjected to hot-melt extrusion (HME). The principle of this method is pushing material through a nozzle at an elevated temperature and pressure, which ensures obtaining a product having uniform density and shape. HME is conducted in an extruder that consists of a filling device, a shaft (screw) that ensures movement of the material through the heated-up space and an extruding head. The dwell time of the mixture of the polymer with nilotinib hydrochloride of form 17 in the extruder is less than 10 minutes, preferably 1 to 5 minutes. The extrusion is carried out at a temperature of 135 to 235°C, preferably at a temperature of 135 to 200°C, more preferably 165 to 215°C, more preferably 165 to 200°C and most preferably at a temperature of 185 ± 10°C.

As the source of nilotinib hydrochloride, the crystalline form T17 is used. This form was first prepared and characterized in the patent application WO2010054056.

Any polymer approved for pharmaceutical use and exhibiting thermoplastic characteristics can be essentially used to create the polymeric matrix. With respect to the characteristics of the pharmaceutically active substance and/or the resulting extrudate, some polymers may be more suitable than others in certain cases. Factors influencing the polymer selection comprise e.g. the chemical purity of the active substance, its thermal stability, hygroscopicity etc. Thus, the selection of a suitable polymer or group of polymers can considerably differ and the suitability of its use cannot be guaranteed in advance as the real interaction of the polymer and active substance may not accurately correspond to the current theoretical knowledge.

Suitable polymers comprise e.g. polyalkylene oxides as polyethylene glycol or polypropylene glycol, copolymers of polyalkylene oxides, homopolymers and copolymers of N-vinyllactams, especially of N-vinylpyrrolidone, such as polyvinylpyrrolidone (PVP), especially copolymers of polyvinylpyrrolidone and polyvinyl acetate, homopolymers and copolymers of acrylic acid and its derivatives, homopolymers and copolymers of methacrylic acid and its derivatives, especially methyl methacrylate, cellulose derivatives as methyl cellulose, ethyl cellulose, hydroxymethyl cellulose, hydroxyethyl cellulose (HEC), hydroxypropyl cellulose (HPC), hydroxypropyl methylcellulose (HPMC), carboxymethyl cellulose, starch derivatives etc.

It has been surprisingly found out that for a preferred embodiment of the invention, a suitable polymer is selected from the group consisting of HPMC, PVPK30 (systematically 1-ethenyl-2-pyrrolidone homopolymer) and Kollidon® VA64 (systematically a copolymer of polyvinyl pyrrolidone - polyvinyl acetate in the percentage ratio of 60:40).

The weight ratio of nilotinib and the polymer is 10:1 to 1:1, preferably 2:1.

Another object of the invention is a crystalline dispersion of nilotinib hydrochloride of the form T17 in a polymeric matrix, preferably prepared using the hot-melt extrusion method. In one embodiment, nilotinib is in the form of a crystalline dispersion in a polymer, which is preferably Kollidon VA64. In another embodiment, crystalline nilotinib is in the form of a crystalline dispersion in a polymer, which is preferably HPMC.

The crystalline dispersion of nilotinib hydrochloride of the form T17 in a polymeric matrix, preferably prepared using the hot-melt extrusion method is suitable for the treatment or prevention of Philadelphia-positive (Ph+) chronic myeloid leukaemia and Philadelphia-positive (Ph+) acute lymphoblastic leukaemia.

Another object of the invention is a pharmaceutical composition comprising a crystalline dispersion of nilotinib hydrochloride of the form T17 in a polymeric matrix prepared using the hot-melt extrusion method. The content of nilotinib in the pharmaceutical composition is 50 to 500 mg, which represents 10 to 90% of the total weight of the composition. The content of nilotinib is preferably 150 to 200 mg.

The composition can also comprise one or more pharmaceutically acceptable excipients that are especially used as fillers, binders, lubricants, surfactants, disintegrants, dyes, solvents, antimicrobial substances or taste and olfactory correctors. The composition preferably comprises at least one excipient selected from the group consisting of microcrystalline cellulose, lactose, sodium lauryl sulphate, sodium croscarmellose, lactose, talc and magnesium stearate.

The pharmaceutical composition can virtually be prepared in any solid drug form, e.g. in the form of tablets, capsules, powders, pellets or granules. A preferred drug form is a capsule, tablet or coated tablet. A capsule can be obtained in such a way that extrudate obtained in the above mentioned manner is ground into particles having the size of D(0.9) ≥ 100 µm, preferably to particles having the size of D(0,9) ≤ 0.5 mm, or D(0.9) ≤ 1.0 mm (measured using an image analysis), mixed with pharmaceutically acceptable excipients and subjected to filling into capsules.

### Detailed description of the Invention

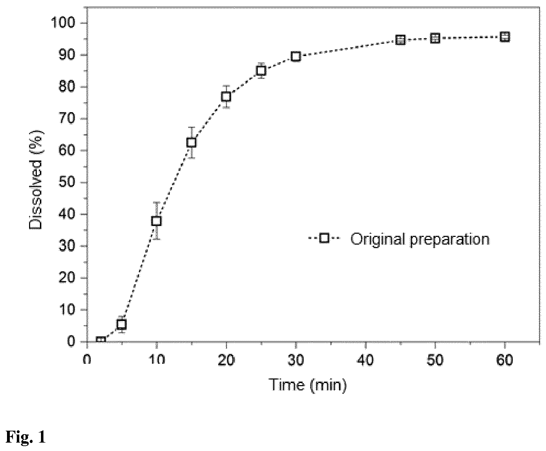

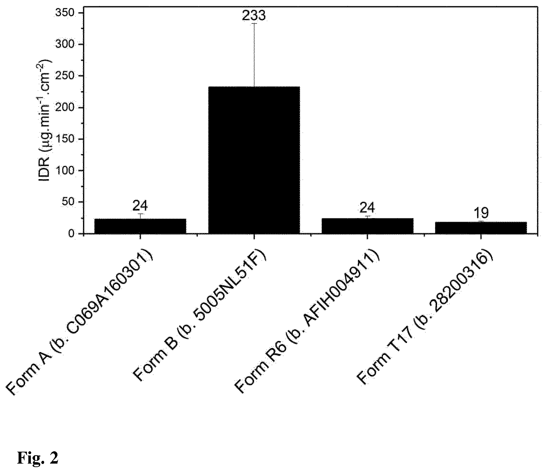

During the development of a solid drug form of crystalline nilotinib, it was necessary to solve adverse physicochemical characteristics of nilotinib of the form T17. The principal factor influencing the quality of the final product is polymorphic instability of the form T17 if this form is exposed to humidity or to an aqueous environment. Dry granulation in combination with suitable selected excipients, especially crospovidone, is able to protect the form T17 during the formulation process and also later during the life cycle of the preparation as described in the patent application WO2017064538. However, it cannot guarantee the required dissolution profile (Fig. 1) because immediate conversion to form A occurs in the dissolution media, which is water. Form A then does not have the required dissolution profile. Figure 2 shows true dissolution of various polymorphic forms of nilotinib hydrochloride. Form B used in the original preparation Tasigna exhibits dissolution that is an order higher than dissolution of the compared form A (described in the patent application WO2007015870), R6 (described in the patent application WO2014174456) and T17 (described in the patent application WO2010054056). However, forms R6 and T17 get immediately converted to form A if these forms are exposed to an aqueous environment. Thus, their true dissolution is distorted and corresponds in fact to the dissolution of form A. Within the preparation of drug forms containing crystalline nilotinib hydrochloride in the polymorphic form T17 or R6 it is therefore necessary to prevent immediate conversion of these forms to form A in an aqueous environment as if immediate conversion to form A occurs, the required dissolution profile of the final preparation cannot be achieved, see Fig. 1.

Thus, none of the methods known from the prior art was suitable for industrial production of a pharmaceutical composition comprising crystalline nilotinib hydrochloride in the polymorphic form T17. However, when looking for a novel preparation method of a pharmaceutical composition comprising nilotinib hydrochloride in the form T17, the authors of the invention surprisingly found out that hot-melt extrusion can be used for its preparation.

This is a special case of using hot-melt extrusion. In fact, it is only the used polymer that is melted while the active ingredient is not melted in it and is evenly distributed in the polymer for the final extrudate to comprise the active ingredient in the original unchanged crystalline form embedded in the polymeric matrix. It has been surprisingly found out that a polymeric matrix consisting of Kollidon VA64 is able to protect the form T17 of nilotinib hydrochloride from a change of the polymorphic form due to surrounding humidity during preparation of the pharmaceutical composition as well as during the life cycle of the product. Further, this polymeric matric can slow down the conversion rate of the form T17 to form A during the dissolution test. Thus, the desired dissolution profile can be achieved.

Nilotinib in the form of crystalline dispersion in a polymeric matrix can be easily formulated with excipients to a pharmaceutical composition. A pharmaceutical formulation prepared this way exhibits high stability, there is no subsequent change of the polymorphic form or degradation of nilotinib and it provides the desired dissolution profile.

### Brief Description of the Drawings

**Fig. 1**: Dissolution of the reference preparation nilotinib hydrochloride (Tasigna, 200 mg) in media at pH 2 (10 mM HCl) and the volume of 900 ml.
**Fig. 2**: Comparison of true dissolutions of selected polymorphic forms of nilotinib hydrochloride.
**Fig. 3**: XRPD characterization of a) crystalline nilotinib of the form T17 (bottom curve), b) extrudate prepared using the procedure of Example 1 at the HME temperature of 185°C and 195°C (top curve).
**Fig. 4**: Dissolution profile of extrudate prepared using the procedure of Example 4 at the HME temperature of 185±10°C, ground through a 1.0 mm sieve. The dissolution profile of the original preparation is also shown here for comparison.

### Examples

The embodiment examples below are only used to illustrate and clarify the invention and are by no means intended to restrict the protection scope, which is only delimited by the wording of the patent claims.

### Example 1

500 g of nilotinib hydrochloride in the polymorphic form T17 was mixed with 250 g of Kollidon VA64 and the resulting mixture was homogenized in a homogenization device. Then, the mixture of the polymer and nilotinib was fed to a hot-melt extruder. The extruder was equipped with five heating segments out of which the first one was set to the temperature of 135°C, the next two ones to the temperature of 185°C and the remaining two to the temperature of 195°C. The amount of the mixture supplied to the extruder was calculated in such a way that the dwell time of the mixture in the extruder should not exceed ten minutes and hot-melt extrusion was carried out. The resulting extrudate was pushed out through a nozzle having the diameter of 0.5 mm, 1.0 mm or 1.5 mm and it was directly cut into particles having the size of 0.5 mm, 1.0 mm or 1.5 mm.

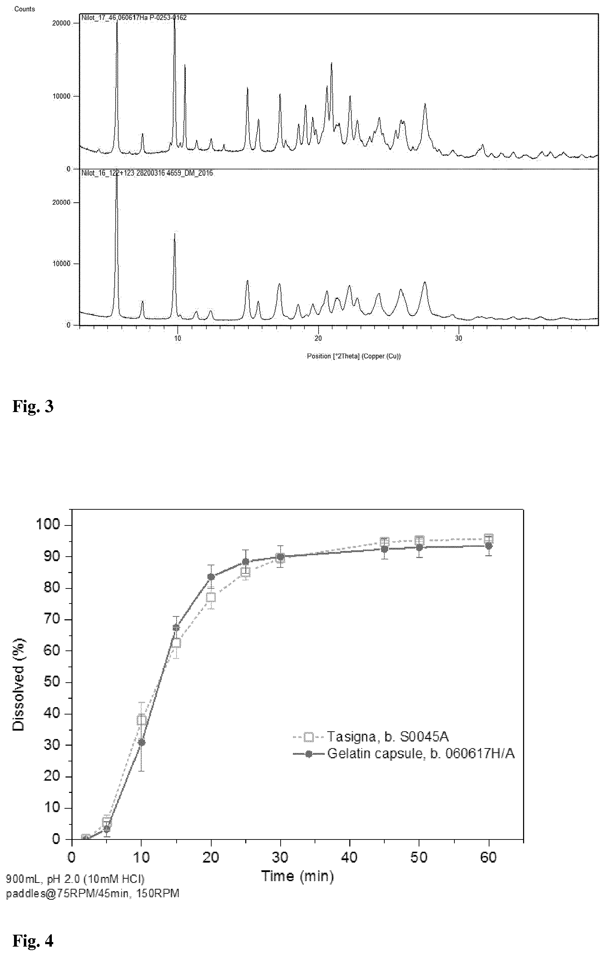

The prepared extrudate was characterized using the X-ray powder diffraction (XRPD) method, see Fig. 3, and the liquid chromatography method, see Table 1.

**Table 1: Profile of impurities of the sample prepared according to Example 1. The abbreviation RRT refers to Relative Retention Time, i.e. the retention time of an impurity.**

| Contents,% | RRT=0.40 | RRT=0.65 | RRT=0.73 | RRT=0.78 | RRT=1.06 | SUM of impurities |
|---|---|---|---|---|---|---|
| 96.8 | 0.00 | 0.00 | 0.06 | 0.00 | 0.07 | 0.13 |

### Example 2

500 g of nilotinib hydrochloride in the polymorphic form T17 was mixed with 250 g of Kollidon VA64 and the resulting mixture was homogenized in a homogenization device. Then, the mixture of the polymer and nilotinib was fed to a hot-melt extruder. The extruder was equipped with five heating segments out of which the first one was set to the temperature of 135°C. The temperature of the other four segments was changed according to Table 2. The amount of the mixture supplied to the extruder was calculated in such a way that the dwell time of the mixture in the extruder should not exceed ten minutes and hot-melt extrusion was carried out. The resulting extrudate was pushed out through a nozzle having the diameter of 0.5 mm, 1.0 mm or 1.5 mm and it was directly cut into particles having the size of 0.5 mm, 1.0 mm or 1.5 mm.

**Table 2: Temperature of the heating segments in the embodiment of Example 2.**

| Experiment | Temperature of heating segments 2 and 3 [°C] | Temperature of heating segments 4 and 5 [°C] | Result |
|---|---|---|---|
| a | 150-170 | 150-170 | Process problems, extruder congestion |
| b | 150-170 | 170-195 | Process problems, extruder congestion |
| c | 170-200 | 180-200 | No process problems, Acceptable polymorphism and contents of impurities |
| d | 200-220 | 200-220 | Higher contents of impurities |

### Example 3

500 g of nilotinib hydrochloride in the polymorphic form T17 was mixed with 250 g of Povidone PVP K30 and the resulting mixture was homogenized in a homogenization device. Then, the mixture of the polymer and nilotinib was fed to a hot-melt extruder. The extruder was equipped with five heating segments out of which the first one was set to the temperature of 150°C and the remaining ones to the temperature of 210°C. The amount of the mixture supplied to the extruder was calculated in such a way that the dwell time of the mixture in the extruder should not exceed ten minutes and hot-melt extrusion was carried out. The resulting extrudate was pushed out through a nozzle having the diameter of 0.5 mm, 1.0 mm or 1.5 mm and it was directly cut into particles having the size of 0.5 mm, 1.0 mm or 1.5 mm.

The prepared extrudate provided an acceptable dissolution profile and polymorphism, but an unacceptable profile of impurities. Hence the final temperature of 210°C or higher is regarded as unsuitable for obtaining a product with an acceptable impurity profile.

### Example 4

Extrudate prepared using the procedure according to Example 1 and ground through a 1.0 mm sieve was mixed with talc, magnesium stearate, sodium lauryl sulphate, anhydrous lactose and sodium croscarmellose. The mixture was homogenized and filled into hard gelatin capsules with the strength of 150 mg or 200 mg of nilotinib. The composition of individual capsules is presented in Table 3 and 4. The dissolution profile of the final preparation with the strength of 200 mg is shown in Figure 4.

**Table 3: Pharmaceutical composition comprising nilotinib with the strength of 150 mg**

| Component | Function | Content |
|---|---|---|
| Extrudate (Nilotinib HCl+Kollidon VA64) | Polymeric matrix with the active ingredient | 240.5 mg (160.3 mg + 80.2 mg) |
| Sodium croscarmellose | Disintegrant | 12.0 |
| Anhydrous lactose | Filler | 48.0 |
| Sodium lauryl sulfate | Surfactant | 1.2 |
| Talc | Glidant | 1.2 |
| Magnesium stearate | Glidant | 0.6 |

**Table 4: Pharmaceutical composition comprising nilotinib with the strength of 200 mg**

| Component | Function | Content |
|---|---|---|
| Extrudate (Nilotinib HCl+Kollidon VA64) | Polymeric matrix with the active ingredient | 320.6 mg (213.7 mg + 106.9 mg) |
| Sodium croscarmellose | Disintegrant | 16.0 |
| Anhydrous lactose | Filler | 64.0 |
| Sodium lauryl sulfate | Surfactant | 1.6 |
| Talc | Glidant | 1.6 |
| Magnesium Stearate | Glidant | 0.8 |

### Example 5

Extrudate prepared using the procedure according to Example 1 and ground through a 1.0 mm sieve was mixed with talc, magnesium stearate, sodium lauryl sulphate, anhydrous lactose and sodium croscarmellose. The mixture was homogenized and filled into hard HPMC capsules with the strength of 150 mg or 200 mg of nilotinib. The composition of individual capsules is presented in Table 5 and 6.

**Table 5: Pharmaceutical composition comprising nilotinib with the strength of 150 mg**

| Component | Function | Content |
|---|---|---|
| Extrudate (Nilotinib HCl+Kollidon VA64) | Polymeric matrix with the active ingredient | 240.5 mg (160.3 mg + 80.2 mg) |
| Sodium croscarmellose | Disintegrant | 12.0 |
| Anhydrous lactose | Filler | 48.0 |
| Sodium lauryl sulfate | Surfactant | 1.2 |
| Talc | Glidant | 1.2 |
| Magnesium stearate | Glidant | 0.6 |

**Table 6: Pharmaceutical composition comprising nilotinib with the strength of 200 mg**

| Component | Function | Content |
|---|---|---|
| Extrudate (Nilotinib HCl+Kollidon VA64) | Polymeric matrix with the active ingredient | 320.6 mg (213.7 mg + 106.9 mg) |
| Sodium croscarmellose | Disintegrant | 16.0 |
| Anhydrous lactose | Filler | 64.0 |
| Sodium lauryl sulfate | Surfactant | 1.6 |
| Talc | Glidant | 1.6 |
| Magnesium Stearate | Glidant | 0.8 |

### Example 6

Extrudate prepared using the procedure of Example 1 and ground through a 1.0 mm sieve was mixed with talc. The mixture was homogenized and filled into hard gelatin capsules with the strength of 150 mg or 200 mg of nilotinib. The composition of individual capsules is presented in Table 7 and 8.

**Table 7: Pharmaceutical composition comprising nilotinib with the strength of 200 mg**

| Component | Function | Content |
|---|---|---|
| Extrudate (Nilotinib HCl+Kollidon VA64) | Polymeric matrix with the active ingredient | 320.6 mg (213.7 mg + 106.9 mg) |
| Talc | Glidant | 3.2 mg |

**Table 8: Pharmaceutical composition comprising nilotinib with the strength of 150 mg**

| Component | Function | Content |
|---|---|---|
| Extrudate (Nilotinib HCl+Kollidon VA64) | Polymeric matrix with the active ingredient | 240.5 mg (160.3 mg + 80.2 mg) |
| Talc | Glidant | 3.2 mg |

### Example 7

Extrudate prepared using the procedure according to Example 1 and ground through a 1.0 mm sieve was mixed with talc, sodium lauryl sulphate and sodium croscarmellose. The mixture was homogenized and filled into hard HPMC capsules with the strength of 150 mg or 200 mg of nilotinib. The composition of individual capsules is presented in Table 9 and 10.

**Table 9: Pharmaceutical composition comprising nilotinib with the strength of 150 mg**

| Component | Function | Content |
|---|---|---|
| Extrudate (Nilotinib HCl+Kollidon VA64) | Polymeric matrix with the active ingredient | 240.5 mg (160.3 mg + 80.2 mg) |
| Sodium croscarmellose | Disintegrant | 12.0 |
| Sodium lauryl sulfate | Surfactant | 1.2 |
| Talc | Glidant | 1.2 |

**Table 10: Pharmaceutical composition comprising nilotinib with the strength of 200 mg**

| Component | Function | Content |
|---|---|---|
| Extrudate (Nilotinib HCl+Kollidon VA64) | Polymeric matrix with the active ingredient | 320.6 mg (213.7 mg + 106.9 mg) |
| Sodium croscarmellose | Disintegrant | 16.0 |
| Sodium lauryl sulfate | Surfactant | 1.6 |
| Talc | Glidant | 1.6 |

### List of analytical methods

### XRPD - X-ray powder diffraction

The diffractograms were obtained by means of a powder X'PERT PRO MPD PANalytical diffractometer, used radiation CuKα (λ = 1.542 Å), excitation voltage: 45 kV, anode current: 40 mA, measured range: 2 - 40° 2θ, increment: 0.02° 2θ with the reflection dwell time of 200 s. The measurements were carried out with a flat sample applied on a Si plate. For the correction of the primary array, 0.02 rad Soller slits, a 10 mm mask and a 1/4° fixed anti-dispersion slit were used. The irradiated area of a sample is 10 mm, programmable divergency slits were used. For the correction of the secondary array, 0.02 rad Soller slits and a 5.0 mm anti-dispersion slit were used.

### HPLC - liquid chromatography

The purity of measured samples was measured with the use of liquid chromatography. The measurement conditions are summarized below:

| | |
|---|---|
| Column: | ACQUITY CSH C18, 1.7 µm, 100 x 2.1 mm (water), or its equivalent |
| Mobile phase: | Component A with component B according to the respective gradient program, see below |
| Component A: | Phosphate buffer pH 3.5 |
| Component B: | Methanol |
| Flow rate: | 0.3 ml/min |
| Injected volume: | 1.0 µl |
| Automatic sampler temperature: | 20°C |
| Column temperature: | 35°C |
| Detection: | UV, 260 nm, sampling rate: At least 5 points a second |

### Gradient program

| Time [min] | Component A [%] | Component B [%] |
|---|---|---|
| 0 | 70 | 30 |
| 6.0 | 10 | 90 |
| 10.0 | 10 | 90 |
| 10.5 | 70 | 30 |
| 13 | 70 | 30 |

### Dissolution

The dissolution profiles were measured using a Sotax AT7 Smart device connected to a Jena Analytik Specord 200 Plus spectrophotometer. The dissolution media had the volume of 900 ml and pH 2.0 (10 mM HCl). Inside the dissolution vessel, there was a paddle stirrer that rotated at the frequency of 75 rpm for the first 45 minutes and then the rotational speed of the stirrer was increased to 150 rpm.

## Claims

1. A method for preparing a crystalline dispersion of nilotinib hydrochloride of the form T17 in a polymeric matrix, ***characterized in that*** it comprises the steps of: i. mixing nilotinib hydrochloride of the form T17 with a polymer, and ii. subjecting the resulting mixture to hot-melt extrusion.

2. The method of preparing according to any of the preceding claims, ***characterized in that*** the polymer is selected from the group consisting of Kollidon VA64, PVP K30 and HPMC.

3. The method of preparing according to any of the preceding claims, ***characterized in that*** the weight ratio of nilotinib hydrochloride of form T17 and the polymer is 10:1 to 1:1, preferably 2:1.

4. The method of preparing according claim 1, ***characterized in that*** the hot-melt extrusion of step ii. is conducted at a temperature of 135 to 200°C.

5. The method of preparing according claim 1 or 2, ***characterized in that*** the hot-melt extrusion is conducted at a temperature of 165 to 200°C, preferably at a temperature of 175 to 195°C.

6. The method of preparing according to any of the preceding claims, ***characterized in that*** the dwell time of the mixture of nilotinib hydrochloride of the form T17 with the polymer in the extruder in step ii. is less than 10 minutes, preferably 1 to 5 minutes.

7. Crystalline dispersion of nilotinib hydrochloride of the form T17 in a polymeric matrix wherein the weight ratio of nilotinib hydrochloride of the form T17 and the polymer is 2:1.

8. The crystalline dispersion of nilotinib hydrochloride of the form T17 in a polymeric matrix according to claim 7 wherein the polymer is selected from the group consisting of Kollidon VA64, PVP K30 and HPMC.

9. The crystalline dispersion of nilotinib hydrochloride of the form T17 in a polymeric matrix according to claim 7 or 8 for the use to treat or prevent Philadelphia-positive (Ph+) chronic myeloid leukaemia and Philadelphia-positive (Ph+) acute lymphoblastic leukaemia.

10. A pharmaceutical composition, ***characterized in that*** it comprises the crystalline dispersion of nilotinib hydrochloride of the form T17 in a polymeric matrix according to claim 7 or 8.

11. The pharmaceutical composition according to claim 10, ***characterized in that*** the content of nilotinib in the form of said polymeric matrix is 50 to 500 mg, preferably 150 to 200 mg.

12. The pharmaceutical composition according to claim 10 to 11, ***characterized in that*** it is in the form of a capsule, tablet or coated tablet.

13. The pharmaceutical composition according to any of claims 10 to 12, ***characterized in that*** it further comprises at least one excipient selected from the group consisting of sodium lauryl sulphate, sodium croscarmellose, lactose, talc and magnesium stearate.
